# EUROPÄISCHE PATENTSCHRIFT

(11) **EP 0 965 330 B1**
(45) Veröffentlichungstag und Bekanntmachung des Hinweises auf die Patenterteilung: **08.06.2005**
(21) Anmeldenummer: 99110757.4
(22) Anmeldetag: 04.06.1999
(51) Int. Cl.: A61K 7/48, A61K 9/107, A61K 47/24, A61K 47/36

(54) **Kosmetische und dermatologische Zubereitungen mit einem Gehalt an Chitosan und Phospholipiden**
Cosmetic and dermatolic compositions containing chitosan and phospholipids
Compositions cosmétiques et dermatologiques contenant du chitosane et des phospholipides

(30) Priorität: 13.06.1998 DE 19826503
(43) Veröffentlichungstag der Anmeldung: 22.12.1999
(73) Patentinhaber: Beiersdorf Aktiengesellschaft, 20245 Hamburg (DE)
(72) Erfinder: Kröpke, Rainer, 22869 Schenefeld (DE); Schneider, Günther, Dr., 22607 Hamburg (DE)

(56) Entgegenhaltungen:
- EP-A- 0 771 566
- FR-A- 2 667 072
- US-A- 5 518 736
- PATENT ABSTRACTS OF JAPAN vol. 098, no. 001, 30. Januar 1998 (1998-01-30) & JP 09 241152 A (SUNSTAR INC), 16. September 1997 (1997-09-16)

## Beschreibung

Die vorliegende Erfindung betrifft kosmetische und dermatologische Zubereitungen mit einem Gehalt an bestimmten Chitosanen und Phospholipiden.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letzlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum corneum), den für die Barrierefunktion bedeutenden Teil darstellt. Das heute in der Fachwelt anerkannte Hautmodell von Elias (*P. M. Elias, Structure and Function of the Stratum Corneum Permeability Barrier, Drug Dev. Res.* ***13****, 1988, 97-105*) beschreibt die Hornschicht als Zwei-Komponenten-System, ähnlich einer Ziegelsteinmauer (Ziegelstein-Mörtel-Modell). In diesem Modell entsprechen die Hornzellen (Korneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Die vorliegende Erfindung betrifft in einer besonderen Ausführungsform kosmetische oder pharmazeutische Zubereitungen mit vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

Chitosan stellt ein partiell deacyliertes Chitin dar. Dieses Biopolymer hat u.a. filmbildende Eigenschaften und zeichnet sich durch ein seidiges Hautgefühl aus. Von Nachteil ist jedoch seine starke Klebrigkeit auf der Haut, die insbesondere - vorübergehend - während der Anwendung auftritt. Entsprechende Zubereitungen können dann im Einzelfalle nicht vermarktungsfähig sein, da sie vom Verbraucher nicht akzeptiert bzw. negativ beurteilt werden.

Es ist zwar bekannt, durch Hinzufügen bestimmter Substanzen, beispielsweise einiger ausgewählter Puderrohstoffe, insbesondere Talkum, dieses Klebrigkeitsgefühl oder auch Schmierigkeitsgefühl zu reduzieren. Davon abgesehen, daß dieses nur selten vollständig gelingt, wird durch einen solchen Zusatz auch die Viskosität des betreffenden Produktes verändert und die Stabilität verringert.

Aufgabe war daher, all diesen den Nachteilen des Standes der Technik Abhilfe zu schaffen. Insbesondere sollten Produkte mit verringerter Klebrigkeit bzw. Schmierigkeit zur Verfügung gestellt werden. Produkte auf dem Gebiete der pflegenden Kosmetik, der dekorativen Kosmetik und der pharmakologischen Galenik sollten gleichermaßen von den geschilderten Nachteilen des Standes der Technik befreit werden.

Weiterhin war es eine Aufgabe der Erfindung, kosmetische Grundlagen für kosmetische Zubereitungen zu entwickeln, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

Chitosan ist gekennzeichnet durch folgende Strukturformel: 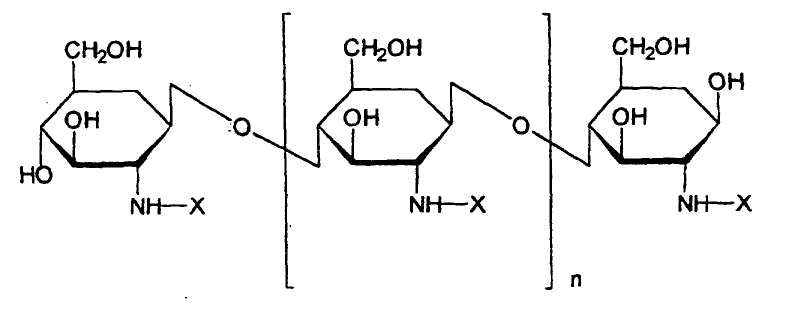 dabei nimmt n Werte bis zu ca. 10.000 an, X stellt entweder den Acetylrest oder Wasserstoff dar. Chitosan entsteht durch Deacetylierung und teilweise Depolymerisation (Hydrolyse) von Chitin, welches durch die Strukturformel 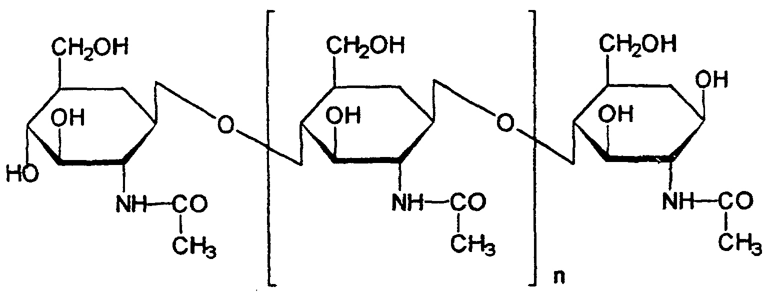 gekennzeichnet ist. Chitin ist wesentlicher Bestandteil des Ektoskeletts ['o χ 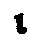 των = grch.: der Panzerrock] der Gliederfüßer (z.B. Insekten, Krebse, Spinnen) und wird auch in Stützgeweben anderer Organismen (z.B. Weichtiere, Algen, Pilze) gefunden.

Chitosan ist ein in der Haarpflege bekannter Rohstoff. Es eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H.P.Fiedler, "Lexikon der Hilfsstoffe für Pharmazie, Kosmetik und angrenzende Gebiete", dritte Auflage 1989, Editio Cantor, Aulendorf, S. 293, Stichwort "Chitosan".

Im Bereich von etwa pH <6 ist Chitosan positiv geladen und dort auch in wäßrigen Systemen löslich. Es ist nicht kompatibel mit anionischen Rohstoffen. Daher bietet sich zur Herstellung chitosanhaltiger Öl-in-Wasser-Emulsionen der Einsatz nichtionischer Emulgatoren an. Diese sind an sich bekannt, beispielsweise aus der EP-A 776 657. Die darin aufgeführten Emulsionen (z.B. mit dem O/W-Emulgator Cetylstearylglucosid im Gemisch mit Cetylstearylalkohol) haben jedoch hinsichtlich ihrer sensorischen Qualität gewisse Nachteile.

Ziel der Erfindung war es daher ferner, chitosanhaltige Zubereitungen, insbesondere Emulsionen, insbesondere O/W-Emulsionen, zur Verfügung zu stellen, die stabil sind, sowohl fließfähig als auch cremeartig formulierbar sind, sehr gute kosmetische Eigenschaften besitzen, insbesondere was die Klebrigkeit betrifft, eine sehr gute Hautverträglichkeit sowie Hautpflegeleistung aufweisen.

Erstaunlicherweise werden diese Aufgaben gelöst durch kosmetische oder dermatologische Zubereitungen, welche
(a) Chitosan eines mittleren Molekulargewichtes von 100.000 bis 1.000.000 g/mol und einem Deacylierungsgrad von 10 bis 99% und
(b) ein oder mehrere Phospholipide enthalten.

Die Aufgaben werden ferner überraschend gelöst durch die Verwendung von Kombinationen aus
(a) Chitosan eines mittleren Molekulargewichtes von 10.000 bis 2.000.000 g/mol und einem Deacylierungsgrad von 10 bis 99% und
(b) ein oder mehrere Phospholipide zur Erstellung nicht-klebriger kosmetischer Zubereitungen, insbesondere O/W-Emulsionen oder zur Verminderung der Klebrigkeit von kosmetischer Zubereitungen, insbesondere O/W-EMulsionen.

Die erfindungsgemäßen Zubereitungen zeichnen sich durch erhöhte Stabilität aus, insbesondere wenn sie in Form von Emulsionen, vorteilhaft O/W-Emulsionen, vorliegen. Auch erhöht die Zugabe von Chitosanen und einem oder mehreren Phospholipiden die Stabilität von Emulsionen, insbesondere O/W-Emulsionen.

Die erfindungsgemäßen Zubereitungen sind sowohl fließfähig als auch cremeartig formulierbar, besitzen sehr gute kosmetische Eigenschaften, insbesondere was die Klebrigkeit betrifft, und weisen eine sehr gute Hautverträglichkeit sowie Hautpflegeleistung auf.

Die EP 0 771 566 beschreibt die Stabilisierung von Kolloidalen Systemen mit Hilfe von ionischen Lipid-Polysaccharid-Komplexen. Die FR 2 667 072 beschreibt temäre Komplexe aus Chitosan, lonen und Lipiden. Die JP 09241152 beschreibt O/W-Emulsionen enthaltend Chitosan. Diese Schriften konnten jedoch nicht den Weg zur vorliegenden Erfindung weisen.

Erfindungsgemäß bevorzugt sind Chitosane mit einem Deacetylierungsgrad > 25 % , insbesondere > 55 bis 99% % [bestimmt mittels ¹H-NMR]).

Erfindungsgemäß enthalten kosmetische oder dermatologische Lichtschutzzubereitungen 0,01 bis 50 Gew.-%, vorteilhaft 0,1 bis 10 Gew.-%, ganz besonders bevorzugt 0,25 bis 2,5 Gew.-% Chitosane.

Die Einarbeitung des Chitosans erfolgt in der Regel dadurch, daß eine Suspension von Chitosan, insbesondere von micronisiertem Chitosan, in Wasser mit Hilfe von organischen oder anorganischen Säuren auf einen pH-Wert von ca. 3,5 - 5,5 gestellt, wobei - in der Regel durch Rühren - eine Lösung des Chitosans erhalten wird. Die so erhältlichen klaren Lösungen zeichnen sich beispielsweise als 2 Gew. -%ige Lösung von Chitosan in 1,2% Milchsäure (90%ige wäßrige Lösung) durch eine Viskosität nach Viskotester-VT02 (Haake) aus, die im Bereich von 100 bis 10.000 mPas, vorzugsweise von 200 bis 5.000 mPas liegt.

Vorteilhaft kann beispielsweise Milchsäure verwendet werden, es ist aber ebenfalls von Vorteil, andere Säuren verwendet werden, die lösliche Chitosansalze ergeben, wie z.B. Phosphorsäue, Essigsäure, Ascorbinsäure (diese vorzugsweise unter Verwendung eines Schutzgases), Salzsäure, Glykolsäure, Salpetersäure, 2-Pyrrolidon-5-carbonsäure, Apfelsäure, Salicylsäure, Benzoësäure.
Phospholipide sind Phosphorsäuredi-, oder -monoester, die wegen ihrer fettähnlichen Löslichkeitseigenschaften aufgrund der lipophilen und hydrophilen Komponenten zu den Lipiden gerechnet werden und im Organismus als Membranlipide am Aufbau von Schichten-Strukturen, den Membranen, beteiligt sind.

Phosphatidsäuren sind Glycerinderivate, die in 1-sn- und 2-Stellung mit Fettsäuren (1-sn-Position: meist gesättigt, 2-Position: meist ein- oder mehrfach ungesättigt), an Atom 3-sn dagegen mit Phosphorsäure verestert sind und durch die allgemeine Strukturformel 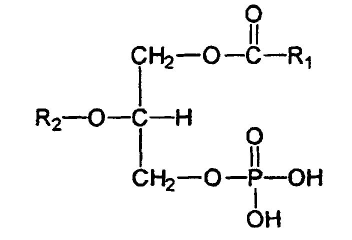 gekennzeichnet.

In den in menschlichem oder tierischem Gewebe vorkommenden Phosphatidsäuren ist der Phosphatrest meist verestert mit Aminoalkoholen wie Cholin (Lecithin = 3-sn-Phosphatidylcholin) oder 2-Aminoethanol (Ethanolamin) bzw. L-Serin (Kephalin = 3-sn-Phosphatidylethanolamin bzw. sn-Phosphatidyl-L-serin), mit myo-Inosit zu den in Geweben häufigen Phosphoinositiden [1-(3-sn-Phosphatidyl)-D-myo-inositen], mit Glycerin zu Phosphatidylglycerinen.

Lecithine sind durch die allgemeine Strukturformel 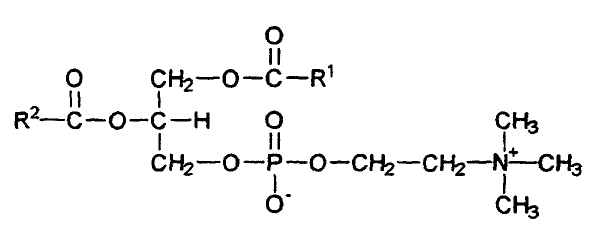 gekennzeichnet, wobei R und R² typischerweise unverzweigte aliphatische Reste mit 15 oder 17 Kohlenstoffatomen und bis zu 4 cis-Doppelbindungen darstellen.

Cardiolipine (1,3-Bisphosphatidylglycerine) sind Phospholipide aus zwei über Glycerin verknüpften Phosphatidsäuren.
Lysophospholipide werden erhalten, wenn aus Phospholipiden ein Acylrest durch Phospholipase A abgespalten wird (z.B. Lysolecithine).

Lysophospholipide sind gekennzeichnet durch die allgemeine Strukturformel

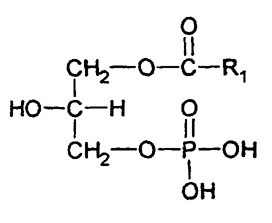

Lysolecithine beispielsweise sind gekennzeichnet durch die allgemeine Strukturformel 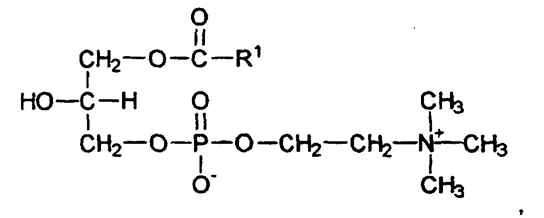 wobei R und R² typischerweise unverzweigte aliphatische Reste mit 15 oder 17 Kohlenstoffatomen und bis zu 4 cis-Doppelbindungen darstellen.

Zu den Phospholipide werden auch Plasmalogene gezählt, in denen statt einer Fettsäure in 1-Stellung ein Aldehyd (in Form eines Enolethers) gebunden ist; die den Phosphatidylcholinen entsprechenden O-1-sn-Alkenyl-Verb. z. B. heißen Phosphatidalcholine.

Den Phosphosphingolipiden liegt als Grundstruktur das Sphingosin oder auch das Phytosphingosin zugrunde, welche sich durch folgende Strukturformeln auszeichnen:

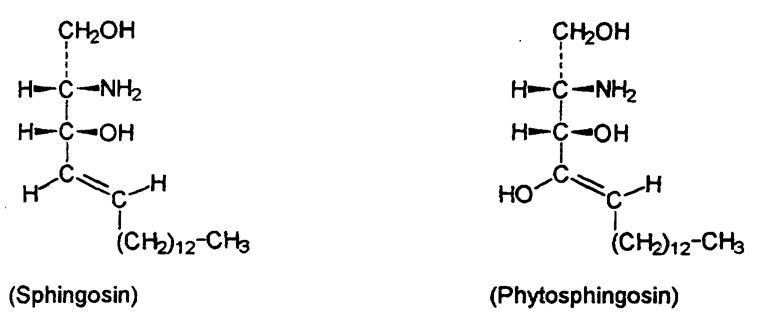

Abwandlungen von Sphingolipiden zeichnen sich beispielsweise aus durch die allgemeine Grundstruktur 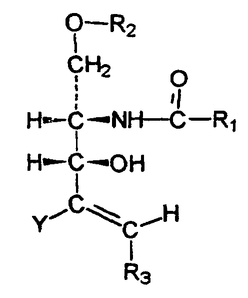 bei welcher R₁ und R₃ unabhängig voneinander gesättigte oder ungesättigte, verzweigte oder unverzweigte Alkylreste von 1 bis 28 Kohlenstoffatomen darstellen, R₂ gewählt wird aus der Gruppe: Wasserstoffatom, gesättigte oder ungesättigte, verzweigte oder unverzweigte Alkylreste von 1 bis 28 Kohlenstoffatomen, Zuckerreste, mit organischen Resten veresterte oder unveresterte Phosphatgruppen, mit organischen Resten veresterte oder unveresterte Sulfatgruppen und Y entweder ein Wasserstoffatom, eine Hydroxygruppe oder einen anderen hetero-funktionellen Rest darstellt.

### Sphingophospholipide:

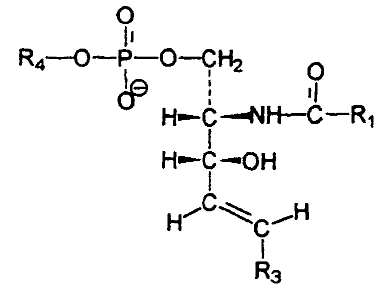

R₁ und R₃ stellen Alkylreste dar, R₄ stellt einen Organylrest dar.

Sphingomyeline sind organylphosphorylierte Sphingolipide des Typs

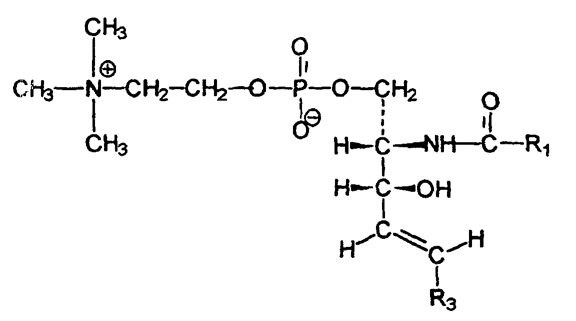

Bevorzugte Phospholipide sind Lecithine. Vorteilhaft zu verwendende Lecithintypen werden gewählt aus Rohlecithinen, welche entölt und/oder fraktioniert und/oder sprühgetrocknet und/oder acetyliert und/oder hydrolysiert und/oder hydriert wurden.

Erfindungsgemäß vorteilhaft zu verwendende Phospholipide sind beispielsweise kauflich zu erwerben unter den Handelsbezeichnungen Phospholipon 25 (Nattermann), Emulmetik 120 (Lucas Meyer), Stempur E (Stern), Stempur PM (Stern), Nathin 3KE (Stern).

Die Menge der Phospholipide (eine oder mehrere Verbindungen) in den Zubereitungen beträgt vorzugsweise 0,001 bis 30 Gew.-%, besonders bevorzugt 0,05 - 20 Gew.-%, insbesondere 0,5 - 5% Gew.-%, bezogen auf das Gesamtgewicht der Zubereitung.

Erfindungsgemäß ist es möglich und vorteilhaft, den Anteil der Ölphase der erfindungsgemäßen Zubereitungen im Bereich von 0,01 bis 99 Gew.-%, bezogen auf das Gesamtgewicht der Zubereitungen, frei zu wählen.

Als Grundbestandteile der erfindungsgemäßen Zubereitungen können verwendet werden:
- Wasser oder wäßrige Lösungen
- wäßrige ethanolische Lösungen
- natürliche Öle und/oder chemisch modifizierte natürliche Öle und/oder synthetische Öle;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet.

Die Ölphase der Emulsionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Camaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), Syncrowax HGLC (C₁₆₋₃₆-Fettsäuretriglycerid) und Syncrowax AW 1C (C₁₈₋₃₆-Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Erfindungsgemäß können die Fett- und/oder Wachskomponenten sowohl einzeln als auch im Gemisch vorliegen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Erfindungsgemäße Emulsionen im Sinne der vorliegenden Erfindung, z.B. in Form einer Hautschutzcreme, einer Hautlotion, einer kosmetischen Milch, beispielsweise in Form einer Sonnenschutzcreme oder einer Sonnenschutzmilch, sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist ebenfalls von Vorteil, von den erfindungsgemäßen Eigenschaften in Form von dekorativen Kosmetika (Make-Up-Formulierungen) Gebrauch zu machen.

Günstig sind auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoesäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:
- 2-Phenylbenzimidazol-5-sulfonsäure und deren Salze, z.B. Natrium-, Kaliumoder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bomylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl) propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Es ist vorteilhaft, den Zubereitungen im Sinne der vorliegenden Erfindung weitere antiirritative oder antientzündliche Wirkstoffe zuzugeben, insbesondere Batylalkohol (α-Octadecylglycerylether), Selachylalkohol (α-9-Octadecenylglycerylether), Chimylalkohol (α-Hexadecylglycerylether), Bisabolol und/oder Panthenol.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Zubereitungen gemäß der vorliegenden Erfindung können auch Verwendung als Grundlage für kosmetische oder dermatologische Desodorantien bzw. Antitranspirantien finden. Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure.

Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor 2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung zur Behandlung und Pflege der Haare, die den erfindungsgemäß verwendeten Wirkstoff enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Die Wasserphase der kosmetischen Zubereitungen im Sinne der vorliegenden Erfindung kann auch Gelcharakter aufweisen, die neben einem wirksamen Gehalt am erfindungsgemäß eingesetzten Substanzen und dafür üblicherweise verwendeten Lösungsmitteln, bevorzugt Wasser, noch weitere organische Verdickungsmittel, z.B. Gummiarabikum, Xanthangummi, Natriumalginat, Stärke und Stärkederivate (z.B. Distärkephosphat), Cellulose, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise organisch modifizierte oder auch unmodifizierte Hectorite, Bentonite, oder dergleichen, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Ferner kann es von Vorteil sein, Zubereitungen gemäß der Erfindung grenz- bzw. oberflächenaktive Agentien zuzufügen, beispielsweise kationische Emulgatoren wie insbesondere quaternäre Tenside.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft ist auch, kationische Polymere (z.B. Jaguar C 162 [Hydroxypropyl Guar Hydroxypropyltrimonium Chloride] bzw. modifizierten Magnesiumaluminiumsilikaten (z.B. Quaternium-18-Hectorit, welches z. B. unter der Handelsbezeichnung Bentone® 38 bei der Firma Rheox erhältlich ist, oder Stearalkonium Hectorit, welches z. B. unter der Handelsbezeichnung Softisan® Gel bei der Hüls AG erhältlich ist), einzusetzen.

Erfindungsgemäße Zubereitungen können vorteilhaft auch Ölverdickungsmittel enthalten, um die taktilen Eigenschaften der Emulsion und die Stiftkonsistenz zu verbessern. Vorteilhafte Ölverdickungsmittel im Sinne der vorliegenden Erfindung sind beispielsweise weitere Feststoffe, wie z. B. hydrophobe Siliciumoxide des Typs Aerosil®, welche von der Degussa AG erhältlich sind. Vorteilhafte Aerosil®-Typen sind beispielsweise Aerosil® OX50, Aerosil® 130, Aerosil® 150, Aerosil® 200, Aerosil® 300, Aerosil® 380. Aerosil® MOX 80, Aerosil® MOX 170, Aerosil® COK 84, Aerosil® R 202, Aerosil® R 805, Aerosil® R 812, Aerosil® R 972, Aerosil® R 974 und/oder Aerosil® R976.

Ferner sind auch sogenannte Metallseifen (d. h. die Salze höherer Fettsäuren mit Ausnahme der Alkalisalze) vorteilhafte Ölverdickungsmittel im Sinne der vorliegenden Erfindung, wie beispielsweise Aluminium-Stearat, Zink-Stearat und/oder Magnesium-Stearat.

Ebenfalls vorteilhaft ist, Zubereitungen gemäß der Erfindung amphotere bzw. zwitterionischen Tensiden (z.B. Cocoamidopropylbetain) und Moisturizem (z.B. Betain) zuzusetzen. Vorteilhaft zu verwendende amphotere Tenside sind beispielsweise Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat, N-Alkylaminosäuren, beispielsweise Aminopropylalkylglutamid, Alkylaminopropionsäure, Natriumalkylimidodipropionat und Lauroamphocarboxyglycinat.

Die Menge der ober- bzw. grenzflächenaktiven Substanzen (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Erfindungsgemäße Zubereitungen können auch Wirkstoffe (eine oder mehrere Verbindungen) enthalten, welche gewählt werden aus der Gruppe: Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter. Vorteilhaft ist es auch, die Wirkstoffe aus der Gruppe der rückfettenden Substanzen zu wählen, beispielsweise Purcellinöl, Eucerit® und Neocerit®.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew-%, bezogen auf das Gesamtgewicht der Zubereitung.

Die nachfolgenden Beispiele sollen die vorliegende Erfindung verdeutlichen.

### Herstellungsbeispiel Chitosanlösung:

| | Gew.-% |
|---|---|
| Chitosan | 2,0 |
| Milchsäure (90%ige wäßrige Lösung) | 1,2 |
| Wasser | ad 100,00 |
| pH-Wert ca. | 4,5 |

### Beispiel 1 (O/W-Emulsion):

| | Gew.-% |
|---|---|
| Sonnenblumenkernöl | 5,00 |
| Chitosanlösung gem. Herstellungsbeispiel | 50,00 |
| Lecithin | 1,00 |
| Glycerin | 3,00 |
| Parfüm, Konservierungsmittel, Farbstoffe, Antioxidantien | q.s. |
| Wasser | ad 100,00 |
| pH-Wert | ca. 4,5 |

### Beispiel 2 (O/W-Emulsion):

| | Gew.-% |
|---|---|
| Weizenkeimöl | 5,0 |
| Chitosanlösung gem. Herstellungsbeispiel | 50,0 |
| Lecithin | 1,0 |
| Distärkephosphat | 1,0 |
| Glycerin | 3,0 |
| Parfüm, Konservierungsmittel, Farbstoffe, Antioxidantien | q.s. |
| pH-Wert | ca. 4,5 |
| Wasser | ad. 100,0 |

### Beispiel 3 (O/W-Emulsion):

| | Gew.-% |
|---|---|
| Jojobaöl | 3,00 |
| Caprylic/Capric Triglyceride | 3,00 |
| Dimethicon | 0,50 |
| Dimethiconol | 0,10 |
| Cyclomethicon | 2,00 |
| Dimeticoncopolyol | 0,20 |
| Chitosanlösung gem. Herstellungsbeispiel | 50,00 |
| Lecithin | 2,00 |
| Tocopherolacetat | 0,50 |
| Panthenol | 0,50 |
| Biotin | 0,10 |
| Glycerin | 3,00 |
| 1,3 Butylenglycol | 1,50 |
| Serine | 0,20 |
| Parfüm, Konservierungsmittel, Farbstoffe, Antioxidantien | q.s. |
| Wasser | ad 100,00 |
| pH-Wert | ca. 4,5 |

### Beispiel 4 (O/W-Emulsion):

| | Gew.-% |
|---|---|
| Dimethicon | 2,00 |
| Cyclomethicon | 2,00 |
| Chitosanlösung gem. Herstellungsbeispiel | 50,00 |
| Lecithin | 1,50 |
| Distarch Phosphate | 1,00 |
| Glycerin | 3,00 |
| Betain | 2,00 |
| Parfüm, Konservierungsmittel, Farbstoffe, Antioxidantien | q.s. |
| Wasser | ad 100,00 |
| pH-Wert | ca. 4,5 |

### Beispiel 5 (O/W-Emulsion):

| | Gew.-% |
|---|---|
| Sonnenblumenkernöl | 12,00 |
| Chitosanlösung gem. Herstellungsbeispiel | 75,00 |
| Lecithin | 4,00 |
| Glycerin | 3,00 |
| Parfüm, Konservierungsmittel, Farbstoffe, Antioxidantien | q.s. |
| Wasser | ad 100,00 |
| pH-Wert | ca. 4,5 |

### Beispiel 6 (O/W-Emulsion):

| | Gew.-% |
|---|---|
| Saffloröl | 5,00 |
| Chitosanlösung gem. Herstellungsbeispiel | 50,00 |
| Lecithin | 1,00 |
| Glycerin | 5,00 |
| Distärkephosphat | 1,00 |
| Parfüm, Konservierungsmittel, Farbstoffe, Antioxidantien | q.s. |
| Wasser | ad 100,00 |
| pH-Wert | ca. 4,5 |

### Beispiel 7 (O/W-Emulsion):

| | Gew.-% |
|---|---|
| Chitosanlösung gem. Herstellungsbeispiel | 50,00 |
| Octyldodecanol | 2,00 |
| Squalan | 2,00 |
| Paraffinum liquidum | 1,00 |
| Hydriertes Polysiobuten | 1,00 |
| Lecithin | 2,00 |
| 4-(tert.-Butyl)-4-methoxydibenzoylmethan | 2,00 |
| Octylmethoxycinnamat | 2,50 |
| 4-Methyl-Benzylidencampher | 2,50 |
| Tris-[anilino-(p-carbo-2'-ethyl-1'-hexyloxy)]-triazin | 1,50 |
| Titandioxid | 2,00 |
| Glycerin | 3,00 |
| Parfüm, Konservierungsmittel, Farbstoffe, Antioxidantien | q.s. |
| Wasser | ad 100,00 |
| pH-Wert | ca. 4,5 |

### Beispiel 8 (O/W-Emulsion):

| | Gew.-% |
|---|---|
| Chitosanlösung gem. Herstellungsbeispiel | 50,00 |
| Octyldodecanol | 1,00 |
| C₁₂₋₁₅-Alkyl Benzoate | 1,00 |
| Squalan | 1,00 |
| Lecithin | 1,50 |
| Glycerin | 3,00 |
| Parfüm, Konservierungsmittel, Farbstoffe, Antioxidantien | q.s. |
| Wasser | ad 100,00 |
| pH-Wert | ca. 4,5 |

### Beispiel 9 (O/W-Emulsion):

| | Gew.-% |
|---|---|
| Chitosanlösung gem. Herstellungsbeispiel | 50,00 |
| Octyldodecanol | 1,00 |
| Dicaprylylether | 1,00 |
| Squalan | 1,00 |
| Paraffinum liquidum | 1,00 |
| Hydriertes Polysiobuten | 1,00 |
| Glycerylstearatcitrat | 0,40 |
| Cetylstearylalkohol | 0,20 |
| Lecithin | 1,50 |
| Glycerin | 3,00 |
| Parfüm, Konservierungsmittel, Farbstoffe, Antioxidantien | q.s. |
| Wasser | ad 100,00 |
| pH-Wert | ca. 4,5 |

### Beispiel 10 (O/W-Emulsion):

| | Gew.-% |
|---|---|
| Chitosanlösung gem. Herstellungsbeispiel | 50,00 |
| Distearyldimethylammoniumchlorid | 2,00 |
| Squalan | 2,00 |
| Paraffinum liquidum | 2,00 |
| Hydriertes Polysiobuten | 2,00 |
| Lecithin | 1,50 |
| Glycerin | 3,00 |
| Parfüm, Konservierungsmittel, Farbstoffe, Antioxidantien | q.s. |
| Wasser | ad 100,00 |
| pH-Wert | ca. 4,5 |

### Beispiel 11 (W/O-Emulsion):

| | Gew-% |
|---|---|
| PEG-7-hydriertes Ricinusöl | 4,00 |
| Wollwachsalkohol | 1,50 |
| Chitosanlösung gem. Herstellungsbeispiel | 0,20 |
| Squalan | 1,00 |
| Lecithin | 0,20 |
| Bienenwachs | 3,00 |
| Vaseline | 4,00 |
| Ozokerit | 4,00 |
| Paraffinöl, subliquidum | 8,00 |
| 4-(tert.-Butyl)-4-methoxydibenzoylmethan | 2,00 |
| Octylmethoxycinnamat | 2,50 |
| 4-Methyl-Benzylidencampher | 2,50 |
| Tris-[anilino-(p-carbo-2'-ethyl-1'-hexyloxy)]-triazin | 1,50 |
| Titandioxid | 2,00 |
| Tocopherolacetat | 1,00 |
| Glycerin | 3,00 |
| Natriumchlorid | 0,70 |
| Parfüm, Konservierungsmittel, Farbstoffe, Antioxidantien | q.s. |
| Wasser | ad 100,00 |

### Beispiel 12 (W/O-Emulsion):

| | Gew.-% |
|---|---|
| Polyglyceryl-3 Dioleat | 3,50 |
| Ozokerit | 3,00 |
| Bienenwachs | 2,00 |
| Paraffinöl, subliquidum | 10,00 |
| Cetylstearyloctanoat | 10,00 |
| Chitosanlösung gem. Herstellungsbeispiel | 20,00 |
| Lecithin | 0,40 |
| Glycerin | 5,00 |
| Natriumchlorid | 0,70 |
| Parfüm, Konservierungsmittel, Farbstoffe, Antioxidantien | q.s. |
| Wasser | ad 100,00 |

### Beispiel 13 (W/O-Emulsion)

| | Gew.-% |
|---|---|
| Laurylmethiconcopolyol | 1,50 |
| Cetylmethiconcopolyol | 0,50 |
| Paraffinöl, subliquidum | 20,00 |
| Cylomethicon | 2,00 |
| Dimethicon | 5,00 |
| Chitosanlösung gem. Herstellungsbeispiel | 10,00 |
| Lecithin | 0,20 |
| Glycerin | 10,00 |
| Natriumchlorid | 1,00 |
| Parfüm, Konservierungsmittel, Farbstoffe, Antioxidantien | q.s. |
| Wasser | ad 100,00 |

### Beispiel 14 ("Hydrodispersion"):

| | Gew.-% |
|---|---|
| Sonnenblumenkernöl | 2,50 |
| Chitosanlösung gem. Herstellungsbeispiel | 50,00 |
| Lecithin | 0,10 |
| Distärkephosphat | 1,00 |
| Hectorit | 0,50 |
| Celluloseglycolat | 0,50 |
| Glycerin | 3,00 |
| Parfüm, Konservierungsmittel, Farbstoffe, Antioxidantien | q.s. |
| Wasser | ad 100,00 |
| pH-Wert | ca. 4,5 |

### Beispiel 15 ("Hydrodispersion"):

| | Gew.-% |
|---|---|
| Sonnenblumenkernöl | 2,50 |
| Chitosanlösung gem. Herstellungsbeispiel | 50,00 |
| Lecithin | 0,10 |
| Distärkephosphat | 1,00 |
| Tromethaminmagnesiumaluminiumsilicat | 0,50 |
| Celluloseglycolat | 0,50 |
| Glycerin | 3,00 |
| Parfüm, Konservierungsmittel, Farbstoffe, Antioxidantien | q.s. |
| Wasser | ad 100,00 |
| pH-Wert | ca. 4,5 |

### Beispiel 16 (Emulsions-Make-up)

| | Gew.-% |
|---|---|
| Sonnenblumenkernöl | 7,50 |
| Chitosanlösung gem. Herstellungsbeispiel | 50,00 |
| Lecithin | 2,00 |
| Distärkephosphat | 1,00 |
| Dimethicon | 0,50 |
| Glycerin | 1,50 |
| Magnesiumsilikat | 1,00 |
| Glimmer | 1,00 |
| Eisenoxide | 1,00 |
| Titandioxid | 2,50 |
| Talkum | 5,00 |
| Tapiocastärke | 0,25 |
| Parfüm, Konservierungsmittel, Farbstoffe, Antioxidantien | q.s. |
| Wasser | ad 100,00 |
| pH-Wert | ca. 4,5 |

### Beispiel 17 (Emulsions-Lippenpflegestift)

| | Gew.-% |
|---|---|
| Octyldodecanol | 20,00 |
| Polyglyceryl-3-dioleat | 3,50 |
| Bienenwachs | 12,50 |
| Squalan | 11,00 |
| C₂₀₋₄₀-Alkylstearate | 5,00 |
| Jojobaöl | 10,00 |
| Carnaubawachs | 2,00 |
| Tocopherolacetat | 0,70 |
| Chitosanlösung gem. Herstellungsbeispiel | 5,00 |
| Lecithin | 1,00 |
| Parfüm, Konservierungsmittel, Farbstoffe, Antioxidantien | q.s. |
| Caprylsäure/Caprinsäuretriglyceride | ad 100,0 |

### Beispiel 18 (Alkoholisches Gesichtswasser)

| | Gew.-% |
|---|---|
| Chitosanlösung gem. Herstellungsbeispiel | 50,00 |
| Lecithin | 0,10 |
| Ethanol | ad 100,00 |

### Beispiel 19 (O/W-Emulsion)

| | Gew.-% |
|---|---|
| Sojaöl | 5,00 |
| Chitosanlösung gem. Herstellungsbeispiel | 50,00 |
| Lecithin | 1,00 |
| Hydroxypropylmethylcellulose | 0,50 |
| Ethanol | 5,00 |
| Parfüm, Konservierungsmittel, Farbstoffe, Antioxidantien | q.s. |
| Wasser | ad 100,00 |
| pH-Wert | ca. 4,5 |

### Beispiel 20 (O/W-Emulsion)

| | Gew.-% |
|---|---|
| Sojaöl | 5,00 |
| Chitosanlösung gem. Herstellungsbeispiel | 50,00 |
| Lecithin | 1,00 |
| Isostearylphosphat | 0,25 |
| Ammoniumphosphatid | 0,25 |
| Parfüm, Konservierungsmittel, Farbstoffe, Antioxidantien | q.s. |
| Wasser | ad. 100,00 |
| pH-Wert | ca. 4,5 |

Ein Vergleich der Formulierungen mit der erfindungsgemäß verwendeten Kombinationen aus Chitosan und Phospholipiden (hier verwendet: Lecithin) mit Formulierungen, welche nur einen der Bestandteile Chitosan und Phospholipide enthält, zeigt, daß die erfindungsgemäß verwendeten Kombinationen zu erhöhter Stabilität der zugrundeliegenden Emulsionen (hier: O/W-Emulsionen) führen.

### O/W-Emulsion analog Beispiel 1

| | Gew.-% | | |
|---|---|---|---|
| Sonnenblumenkernöl | 5,00 | 5,00 | 5,00 |
| Chitosanlösung gem. Herstellungsbeispiel | 50,00 | - | 50,00 |
| Lecithin | 1,00 | 1,00 | - |
| Glycerin | 3,00 | 3,00 | 3,00 |
| Parfüm, Konservierungsmittel, Farbstoffe, Antioxidantien | q.s. | q.s. | q.s. |
| Wasser | ad 100,00 | ad 100,00 | ad 100,00 |
| pH-Wert | 4,5 | 4,5 | 4,5 |
| Stabilität der Emulsion: | stabil | instabil | instabil |

## Patentansprüche

1. Kosmetische oder dermatologische Zubereitungen, welche
(a) Chitosan eines mittleren Molekulargewichtes von 100.000 bis 1.000.000 g/mol und einem Deacylierungsgrad von 10 bis 99% und
(b) ein oder mehrere Phospholipide enthalten.

2. Zubereitung nach Anspruch 1, **dadurch gekennzeichnet, dass** das Chitosan einen Deacetylierungsgrad von >25% aufweist.

3. Zubereitung nach einem der vorhergehenden Ansprüche, **dadurch gekennzeichnet, dass** das Chitosan einen Deacetylierungsgrad von >55 bis 99% aufweist.

4. Zubereitung nach einem der vorhergehenden Ansprüche, **dadurch gekennzeichnet, dass** die Menge der Phospholipide (eine oder mehrere Verbindungen) in den Zubereitungen 0,001 bis 30 Gew.%, besonders bevorzugt 0,05 - 20 Gew.-% und insbesondere 0,5 - 5% Gew.-%, bezogen auf das Gesamtgewicht der Zubereitung, beträgt.

5. Zubereitung nach einem der vorhergehenden Ansprüche, **dadurch gekennzeichnet, dass** als Phospholipide Lecithine eingesetzt werden.

6. Zubereitung nach einem der vorhergehenden Ansprüche, **dadurch gekennzeichnet, dass** als Lecithine Lecithintypen eingesetzt werden aus Rohlecithinen, weiche entölt und/oder fraktioniert und/oder sprühgetrocknet und/oder acetyliert und/oder hydrolysiert und/oder hydriert wurden.

7. Zubereitung nach einem der vorhergehenden Ansprüche, **dadurch gekennzeichnet, dass** die Zubereitung eine kosmetische oder dermatologische Lichtschutzzubereitung mit einem Gehalt an Chitosanen von 0,01 bis 50 Gew.-%, vorteilhaft 0,1 bis 10 Gew.-%, ganz besonders bevorzugt 0,25 bis 2,5 Gew.-% darstellt.

8. Verwendung von Kombinationen aus
(a) Chitosan eines mittleren Molekulargewichtes von 10.000 bis 2.000.000 g/mol und einem Deacylierungsgrad von 10 bis 99% und
(b) einem oder mehreren Phospholipiden zur Herstellung nicht-klebriger kosmetischer Zubereitungen, insbesondere O/W-Emulsionen.

9. Verwendung nach Anspruch 8, **dadurch gekennzeichnet, dass** das erfindungsgemäße Chitosan ein mittleres Molekulargewicht von 100.000 bis 1.000.000 g/mol aufweist.

10. Verwendung nach einem der Ansprüche 8 oder 9, **dadurch gekennzeichnet, dass** das Chitosan einen Deacetylierungsgrad von >25% aufweist.

11. Verwendung nach einem der Artsprüche 8-10, **dadurch gekennzeichnet, dass** das Chitosan einen Deacetylierungsgrad von >55 bis 99% aufweist.

12. Verwendung nach einem der Ansprüche 8-11, **dadurch gekennzeichnet, dass** die Menge der Phospholipide (eine oder mehrere Verbindungen) in den Zubereitungen 0,001 bis 30 Gew.-%, besonders bevorzugt 0,05 - 20 Gew.-% und insbesondere 0,5 - 5% Gew.-%, bezogen auf das Gesamtgewicht der Zubereitung, beträgt.

13. Verwendung nach einem der Ansprüche 8-12, **dadurch gekennzeichnet, dass** als Phospholipide Lecithine eingesetzt werden.

14. Verwendung nach einem des Ansprüche 8-13, **dadurch gekennzeichnet, dass** als Lecithine Lecithintypen eingesetzt werden aus Rohlecithinen, welche entölt und/oder fraktioniert und/oder sprühgetrocknet und/oder acetyliert und/oder hydrolysiert und/oder hydriert wurden.

15. Verwendung nach einem der Ansprüche 8-14, **dadurch gekennzeichnet, dass** die Zubereitung eine kosmetische oder dermatologische Lichtschutzzubereitung mit einem Gehalt an Chitosanen von 0,01 bis 50 Gew.-%, vorteilhaft 0,1 bis 10 Gew.-%, ganz besonders bevorzugt 0,25 bis 2,5 Gew.-% darstellt.

16. Verwendung von Kombinationen aus
(a) Chitosan eines mittleren Molekulargewichtes von 10.000 bis 2.000.000 g/mol und einem Deacylierungsgrad von 10 bis 99% und
(b) einem oder mehreren Phospholipiden zur Verminderung der Klebrigkeit von kosmetischen Zubereitungen, insbesondere O/W-EMulsionen.

17. Verwendung nach Anspruch 16, **dadurch gekennzeichnet, dass** das erfindungsgemäße Chitosan ein mittleres Molekulargewicht von 100.000 bis 1.000.000 g/mol aufweist.

18. Verwendung nach einem der Ansprüche 16 oder 17 **dadurch gekennzeichnet, dass** das Chitosan einen Deacetylierungsgrad von >25% aufweist.

19. Verwendung nach einem der Ansprüche 16-18, **dadurch gekennzeichnet, dass** das Chitosan einen Deacetyllerungsgrad von >55 bis 99% aufweist.

20. Verwendung nach einem der Ansprüche 16-19, **dadurch gekennzeichnet, dass** die Menge der Phospholipide (eine oder mehrere Verbindungen) in den Zubereitungen 0,001 bis 30 Ges.-%, besonders bevorzugt 0,05 - 20 Gew.-% und insbesondere 0,5 - 5% Gew.-%, bezogen auf das Gesamtgewicht der Zubereitung, beträgt.

21. Verwendung nach einem der Ansprüche 16-20, **dadurch gekennzeichnet, dass** als Phospholipide Lecithine eingesetzt werden.

22. Verwendung nach einem der Ansprüche 16-21, **dadurch gekennzeichnet, dass** als Lecithine Lecithintypen eingesetzt werden aus Rohlecithinen, welche entölt und/oder fraktioniert und/oder sprühgetrocknet und/oder acetyliert und/oder hydrolysiert und/oder hydriert wurden.

23. Verwendung nach einem der Ansprüche 16-22, **dadurch gekennzeichnet, dass** die Zubereitung eine kosmetische oder dermatologische Lichtschutzzubereitung mit einem Gehalt an Chitosanen von 0,01 bis 50 Gew.-%, vorteilhaft 0,1 bis 10 Gew.-%, ganz besonders bevorzugt 0,25 bis 2,5 Ges.-% darstellt.

## Claims

1. Cosmetic or dermatological preparations which comprise
(a) chitosan of average molecular weight from 100 000 to 1 000 000 g/mol and a degree of deacetylation of from 10 to 99% and
(b) one or more phospholipids.

2. Preparation according to Claim 1, **characterized in that** the chitosan has a degree of deacetylation of > 25%.

3. Preparation according to one of the preceding claims, **characterized in that** the chitosan has a degree of deacetylation of from > 55 to 99%.

4. Preparation according to one of the preceding claims, **characterized in that** the amount of phospholipids (one or more compounds) in the preparations is 0.001 to 30% by weight, particularly preferably 0.05-20% by weight and in particular 0.5-5% by weight, based on the total weight of the preparation.

5. Preparation according to one of the preceding claims, **characterized in that** the phospholipids used are lecithins.

6. Preparation according to one of the preceding claims, **characterized in that** the lecithins used are lecithin types from crude lecithins which have been deoiled and/or fractionated and/or spray-dried and/or acetylated and/or hydrolysed and/or hydrogenated.

7. Preparation according to one of the preceding claims, **characterized in that** the preparation is a cosmetic or dermatological photoprotective preparation with a content of chitosans of from 0.01 to 50% by weight, advantageously 0.1 to 10% by weight, very particularly preferably 0.25 to 2.5% by weight.

8. Use of combinations of
(a) chitosan of average molecular weight from 10 000 to 2 000 000 g/mol and a degree of deacetylation of from 10 to 99% and
(b) one or more phospholipids for preparing non-sticky cosmetic preparations, in particular O/W emulsions.

9. Use according to Claim 8, **characterized in that** the chitosan according to the invention has an average molecular weight of from 100 000 to 1 000 000 g/mol.

10. Use according to one of Claims 8 or 9, **characterized in that** the chitosan has a degree of deacetylation of > 25%.

11. Use according to one of Claims 8-10, **characterized in that** the chitosan has a degree of deacetylation of from > 55 to 99%.

12. Use according to one of Claims 8-11, **characterized in that** the amount of phospholipids (one or more compounds) in the preparations is 0.001 to 30% by weight, particularly preferably 0.05-20% by weight and in particular 0.5-5% by weight, based on the total weight of the preparation.

13. Use according to one of Claims 8-12, **characterized in that** the phospholipids used are lecithins.

14. Use according to one of Claims 8-13, **characterized in that** the lecithins used are lecithin types from crude lecithins which have been deoiled and/or fractionated and/or spray-dried and/or acetylated and/or hydrolysed and/or hydrogenated.

15. Use according to one of Claims 8-14, **characterized in that** the preparation is a cosmetic or dermatological photoprotective preparation with a content of chitosans of from 0.01 to 50% by weight, advantageously 0.1 to 10% by weight, very particularly preferably 0.25 to 2.5% by weight.

16. Use of combinations of
(a) chitosan of average molecular weight from 10 000 to 2 000 000 g/mol and a degree of deacetylation of from 10 to 99% and
(b) one or more phospholipids for reducing the stickiness of cosmetic preparations, in particular O/W emulsions.

17. Use according to Claim 16, **characterized in that** the chitosan according to the invention has an average molecular weight of from 100 000 to 1 000 000 g/mol.

18. Use according to one of Claims 16 or 17, **characterized in that** the chitosan has a degree deacetylation of > 25%.

19. Use according to one of Claims 16-18, **characterized in that** the chitosan has a degree of deacetylation of from > 55 to 99%.

20. Use according to one of Claims 16-19, **characterized in that** the amount of phospholipids (one or more compounds) in the preparations is 0.001 to 30% by weight, particularly preferably 0.05-20% by weight and in particular 0.5-5% by weight, based on the total weight of the preparation.

21. Use according to one of Claims 16-20, **characterized in that** the phospholipids used are lecithins.

22. Use according to one of Claims 16-21, **characterized in that** the lecithins used are lecithin types from crude lecithins which have been deoiled and/or fractionated and/or spray-dried and/or acetylated and/or hydrolysed and/or hydrogenated.

23. Use according to one of Claims 16-22, **characterized in that** the preparation is a cosmetic or dermatological photoprotective preparation with a content of chitosans of from 0.01 to 50% by weight, advantageously 0.1 to 10% by weight, very particularly preferably 0.25 to 2.5% by weight.

## Revendications

1. Préparations cosmétiques ou dermatologiques contenant
(a) un chitosane ayant une masse moléculaire moyenne de 100 000 à 1 000 000 g/mole et un degré de désacylation de 10 à 99 % et
(b) un ou plusieurs phospholipides.

2. Préparation selon la revendication 1, **caractérisée en ce que** le chitosane présente un degré de désacétylation de > 25 %.

3. Préparation selon l'une quelconque des revendications précédentes, **caractérisée en ce que** le chitosane présente un degré de désacétylation de > 55 à 99 %.

4. Préparation selon l'une quelconque des revendications précédentes, **caractérisée en ce que** la quantité des phospholipides (un ou plusieurs composés) dans les préparations va de 0,001 à 30 % en poids, de façon particulièrement préférée de 0,05 à 20 % en poids et en particulier de 0,5 à 5 % en poids, par rapport au poids total de la préparation.

5. Préparation selon l'une quelconque des revendications précédentes, **caractérisée en ce que** des lécithines sont utilisées en tant que phospholipides.

6. Préparation selon l'une quelconque des revendications précédentes, **caractérisée en ce qu'**on utilise en tant que lécithines des types de lécithines choisis parmi des lécithines brutes qui ont été déshuilées et/ou fractionnées et/ou séchées par atomisation et/ou acétylées et/ou hydrolysées et/ou hydrogénées.

7. Préparation selon l'une quelconque des revendications précédentes, **caractérisée en ce que** la préparation représente une préparation photoprotectrice cosmétique ou dermatologique ayant une teneur en chitosanes de 0,01 à 50 % en poids, avantageusement de 0,1 à 10 % en poids, de façon tout particulièrement préférée de 0,25 à 2,5 % en poids.

8. Utilisation d'associations de
(a) chitosane ayant une masse moléculaire moyenne de 10 000 à 2 000 000 g/mole et un degré de désacylation de 10 à 99 % et
(b) un ou plusieurs phospholipides pour la production de préparations cosmétiques non collantes, en particulier d'émulsions H/E.

9. Utilisation selon la revendication 8, **caractérisée en ce que** le chitosane selon l'invention a une masse moléculaire moyenne de 100 000 à 1 000 000 g/mole.

10. Utilisation selon la revendication 8 ou 9, **caractérisée en ce que** le chitosane présente un degré de désacétylation de > 25 %.

11. Utilisation selon l'une quelconque des revendications 8 à 10, **caractérisée en ce que** le chitosane présente un degré de désacétylation de > 55 à 99 %.

12. Utilisation selon l'une quelconque des revendications 8 à 11, **caractérisée en ce que** la quantité des phospholipides (un ou plusieurs composés) dans les préparations va de 0,001 à 30 % en poids, de façon particulièrement préférée de 0,05 à 20 % en poids et en particulier de 0,5 à 5 % en poids, par rapport au poids total de la préparation.

13. Utilisation selon l'une quelconque des revendications 8 à 12, **caractérisée en ce que** des lécithines sont utilisées en tant que phospholipides.

14. Utilisation selon l'une quelconque des revendications 8 à 13, **caractérisée en ce qu'**on utilise en tant que lécithines des types de lécithines choisis parmi des lécithines brutes qui ont été déshuilées et/ou fractionnées et/ou séchées par atomisation et/ou acétylées et/ou hydrolysées et/ou hydrogénées.

15. Utilisation selon l'une quelconque des revendications 8 à 14, **caractérisée en ce que** la préparation représente une préparation photoprotectrice cosmétique ou dermatologique ayant une teneur en chitosanes de 0,01 à 50 % en poids, avantageusement de 0,1 à 10 % en poids, de façon tout particulièrement préférée de 0,25 à 2,5 % en poids.

16. Utilisation d'associations de
(a) chitosane ayant une masse moléculaire moyenne de 10 000 à 2 000 000 g/mole et un degré de désacylation de 10 à 99 % et
(b) un ou plusieurs phospholipides pour éviter la pégosité de préparations cosmétiques, en particulier d'émulsions H/E.

17. Utilisation selon la revendication 16, **caractérisée en ce que** le chitosane selon l'invention a une masse moléculaire moyenne de 100 000 à 1 000 000 g/mole.

18. Utilisation selon la revendication 16 ou 17, **caractérisée en ce que** le chitosane présente un degré de désacétylation de > 25 %.

19. Utilisation selon l'une quelconque des revendications 16 à 18, **caractérisée en ce que** le chitosane présente un degré de désacétylation de > 55 à 99 %.

20. Utilisation selon l'une quelconque des revendications 16 à 19, **caractérisée en ce que** la quantité des phospholipides (un ou plusieurs composés) dans les préparations va de 0,001 à 30 % en poids, de façon particulièrement préférée de 0,05 à 20 % en poids et en particulier de 0,5 à 5 % en poids, par rapport au poids total de la préparation.

21. Utilisation selon l'une quelconque des revendications 16 à 20, **caractérisée en ce que** des lécithines sont utilisées en tant que phospholipides.

22. Utilisation selon l'une quelconque des revendications 16 à 21, **caractérisée en ce qu'**on utilise en tant que lécithines des types de lécithines choisis parmi des lécithines brutes qui ont été déshuilées et/ou fractionnées et/ou séchées par atomisation et/ou acétylées et/ou hydrolysées et/ou hydrogénées.

23. Utilisation selon l'une quelconque des revendications 16 à 22, **caractérisée en ce que** la préparation représente une préparation photoprotectrice cosmétique ou dermatologique ayant une teneur en chitosanes de 0,01 à 50 % en poids, avantageusement de 0,1 à 10 % en poids, de façon tout particulièrement préférée de 0,25 à 2,5 % en poids.
